(19) Europäisches Patentamt
European Patent Office
Office européen des brevets

(11) Publication number: **0 101 570**
**B1**

(12) **EUROPEAN PATENT SPECIFICATION**

(45) Date of publication of patent specification: **27.01.88**

(21) Application number: **83107272.3**

(22) Date of filing: **25.07.83**

(51) Int. Cl.4: **C 07 C 69/734,**
C 07 C 67/317, C 07 C 121/75,
C 07 C 120/00, C 07 C 43/225,
C 07 C 79/35, C 07 C 76/00,
C 07 B 57/00, C 07 C 59/64,
C 07 C 27/02

# (54) Method for the resolution of racemic 2-CP-difluoromethoxy phenyl)-3-methylbutyric acid.

(30) Priority: **23.08.82 US 410805**
**20.09.82 US 420170**
**20.09.82 US 420164**
**20.09.82 US 420169**

(43) Date of publication of application:
**29.02.84 Bulletin 84/09**

(45) Publication of the grant of the patent:
**27.01.88 Bulletin 88/04**

(84) Designated Contracting States:
**BE CH DE FR GB IT LI NL SE**

(56) References cited:
**GB-A-2 017 688**

**T.G. Miller & J.W. Thanassi, J Org. Chem. 25, p. 2009 (1960)**

(73) Proprietor: **AMERICAN CYANAMID COMPANY**
**1937 West Main Street P.O. Box 60**
**Stamford Connecticut 06904-0060 (US)**

(72) Inventor: **Kameswaran, Venkataraman**
**34 Penn-Lyle Road**
**Princeton Junction New Jersey 08550 (US)**
Inventor: **Siddens, Jack Kenneth**
**38B Berrion Avenue**
**Princeton Junction New Jersey 08550 (US)**
Inventor: **Raghu, Sivaraman**
**17 Starlight Drive**
**Norwalk Connecticut 06851 (US)**

(74) Representative: **Wächtershäuser, Günter, Dr.**
**Tal 29**
**D-8000 München 2 (DE)**

**EP 0 101 570 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention herein described relates to a method for the preparation of difluoromethoxyaromatic compounds which are useful as intermediates in the preparation of pyrethroid pesticides. The present invention also relates to a method for the preparation of difluoromethoxyaromatic compounds involving alkylation of a *p*-substituted phenol with excess chlorodifluoromethane at atmospheric or superatmospheric pressure in the presence of a base, water, and an inert water miscible organic solvent or solvent mixture, alone or in the presence of benzyltriethylammonium chloride. Advantageously, it has been found that when the above-said difluoromethoxyaromatic compound contains a side chain acetic acid ester having a chiral center, it is amenable for use in either of the following reactions which involve: (a) hydrolyzing said ester to the corresponding racemic acid and thereafter resolving said racemic acid into its dextrorotatory (+) acid and levorotatory (−) acid isomers and recovering said (+) acid isomer; or (b) hydrolyzing said ester to the corresponding racemic acid, resolving said racemic acid into its d(+) acid and l(−) acid isomers, recovering said d(+) acid for use in the preparation of a pyrethroid pesticide product, esterifying said l(−) acid from the above-said resolution, racemizing the thus-prepared l(−) ester from said esterification and hydrolyzing the same to obtain the racemic acid which is recycled for admixture with the fresh racemic acid prior to resolution.

By way of background, pyrethroid pesticides are disclosed in United States Patent No. 4,199,595 which is incorporated herein by way of reference. Some pyrethroids serve as broad spectrum pesticides which are highly effective as contact and stomach poisons against ixodide ticks and a wide variety of insects, (i.e., Dipterous, Lepidopterous, Coleopterous, and Homopterous insects). In addition, some pyrethroids exhibit extended residual insecticidal activity on plant tissue and are surprisingly effective for the control of ixodidae. They can be used for the protection of animals against attack by both insects and ixodidae when administered to the animals orally or parenterally or applied thereto as a topical insecticidal or acaricidal formulation.

The method as defined in the preamble of claim 1 is known from J. Org. Chem. *25,* 2009 (1960). A dioxane water mixture is used as solvent and the yields are insufficient.

In accordance with the present invention, difluoromethoxyaromatic compounds represented by the following structural formula:

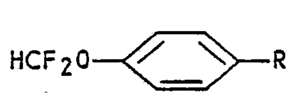

(I)

wherein R is selected from $C_1$—$C_3$ alkyl, halogen, nitro, or is the moiety

$$\begin{array}{l}—CH—R_1\\ \quad|\\ \quad CH(CH_3)_2\end{array}$$

wherein $R_1$ is —CN, —$COOR_2$, OH or $OR_3$, and $R_2$ is $C_1$—$C_8$ alkyl, and $R_3$ is tosyl, mesyl or $C_2$—$C_4$ alkanoyl, can be prepared by the method as claimed in claim 1.

Formula (I) compounds may be prepared by reacting one molar equivalent of a compound of structural formula

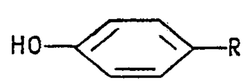

(II)

wherein R is as hereinabove defined, at a temperature ranging from approximately 20 to 40°C, in a solvent mixture, of 2-propanol:acetonitrile or 2-propanol:acetone. The solvent mixture contains the above solvents in 1:1 to 1:3 volume ratios, and said solvent mixture is used in amounts of from 4 to 6 mL per gram of the compound of formula (II) in the initial presence of from approximately 870 to 1740 mol percent of water, with two to three molar equivalents of chlorodifluoromethane added under a pressure of from 0.4 to 2.5 kg $cm^{-2}$. The chlorodifluoromethane is added over a period of time ranging from approximately 30 minutes to three hours and with an aqueous solution of four molar equivalents of an alkali metal hydroxide of sodium or potassium. The concentration of said aqueous solution is such that on completion of the base addition, the total amount of water added in the reaction mixture is of from approximately 1740 to 2610 mol percent, added essentially simultaneously with the chlorodifluoromethane addition over a period of time ranging from approximately one to five hours. On completion of the base addition, the reaction mixture is further maintained at the above pressure and temperature ranges for a period of time from zero to six hours, or until said reaction is essentially complete.

A preferred method for carrying out the above procedure involves the addition to a solution at a temperature of from 30 to 35°C of one molar equivalent of a compound of formula (II) in a solvent mixture of 2-propanol:acetone wherein the volume ratio of said solvent is 1:1 and in the total volume amount of 4 mL per gram of a compound of formula (I) and water, which is added initially at the rate of 1740 mole

percent, is added 3 molar equivalents of chlorodifluoromethane over a time period ranging from 30 minutes to one hour at a pressure of from approximately 0.4 kg $cm^{-2}$ along with initially the simultaneous addition of 4 molar equivalents of an aqueous sodium hydroxide solution which is added over a period of time of approximately one hour to two hours; and, on the completion of the base addition, the total amount of water added is 2610 mol percent, and the reaction is further maintained at the above pressure and temperature range for a period of time of from approximately one hour to two hours.

As indicated above, certain of the compounds of formula (I) are useful and valuable intermediates for the preparation of pyrethroid-type pesticides. For example, compounds of formula (I) wherein R is

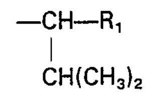

alkyl, may be hydrolyzed to the corresponding racemic acid (III). The racemic acid (III) may then be converted to the acid chloride by heating said acid (III) with thionyl chloride. The thus-prepared acid chloride (IV) is then reacted with *m*-phenoxybenzyl alcohol or α-cyano-*m*-phenoxybenzyl alcohol (V) in the presence of an acid acceptor to yield the pesticidal pyrethroid *m*-phenoxybenzyl or α-cyano-*m*-phenoxybenzyl-2-[*p*-(difluoromethoxy)phenyl]-3-methylbutyrate (VI).

These reactions may be graphically illustrated as follows:

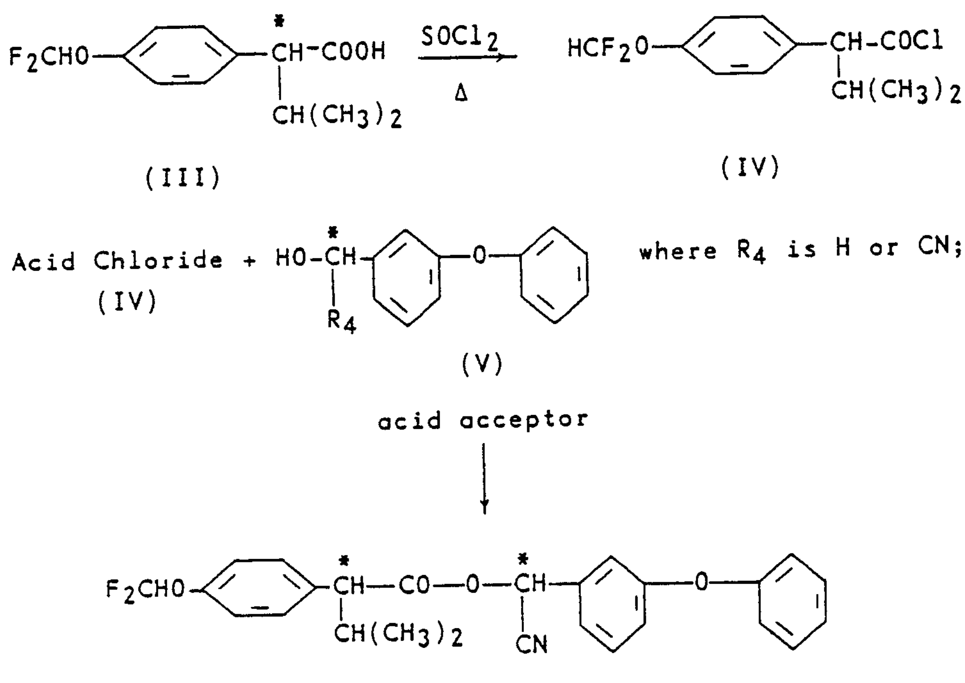


(III) (IV) (IV) (V) (VI)

The pyrethroid of formula (VI) possesses two chiral centers which are indicated by asterisks in the above structure. Therefore, if it is prepared from the unresolved acid of formula (III) and the unresolved α-cyano-*m*-phenoxybenzyl alcohol of formula (V), it will be a mixture of four optical isomers. However, since the insecticidal-pesticidal activity of the pyrethroid of formula (VI) increases twofold when prepared from the resolved dextrorotatory (+) acid of formula (VII) (c.f., United States Patent No. 4,199,595), it is desirable to prepare this pyrethroid using (+)-2-(*p*-difluoromethoxyphenyl)-3-methylbutyric acid (VII).

Thus, the formula (III) racemic acid, i.e., (±)-2-(*p*-difluoromethoxyphenyl)-3-methylbutyric acid, may be resolved by the following method if preparation of the d(+) acid is desired.

One molar equivalent of the racemic acid of formula (III) is dissolved in a water immiscible solvent selected from benzene, toluene, xylene, or mixtures thereof. To the above solution is then added about 40 to 100 mol percent, preferably 50 to 70 mol percent, of (−)-α-phenethylamine, and about 30 to 60 mol percent of an aqueous solution of a base selected from alkali metal hydroxides, carbonates, bicarbonates, and ammonium hydroxide. If so desired, additional water may also be incorporated into the above reaction mixture, above and beyond the amounts already present in said aqueous base. Next, this reaction mixture is stirred and heated at a temperature ranging from about 40°C to the boiling point of said reaction mixture at atmospheric pressure for a period of time sufficient to convert essentially all of the (+) acid present to its (−)-α-phenethylamine salt. The reaction mixture is cooled. The precipitated (−)-α-phenethylamine salt of (+)-2-(*p*-difluoromethoxyphenyl)-3-methylbutyric acid is then removed from the reaction mixture and

purified by standard laboratory procedures (i.e. recrystallization), if so desired.

Finally, the (+)-2-(*p*-difluoromethoxyphenyl)-3-methylbutyric acid is regenerated from the above salt by stirring with a mixture of a water immiscible solvent selected from the group listed above, a dilute acid (i.e., dilute sulfuric or hydrochloric acid used in excess over theory) and additional water if so desired. The thus-regenerated formula (III) dextrorotatory acid is present in the solvent phase and may be isolated therefrom by standard laboratory procedures, or said solution may be used directly for the preparation of the formula (VI) pyrethroid.

Advantageously, this method employs a two-phase solvent system, the use of which affords the desired (+) acid in improved yields when compared to yields obtainable by conventional procedures used to resolve such racemates.

Other resolving agents which may be utilized to good advantage in the above-described process for the isolation of the (+) formula (VII) acid may be selected from the group consisting of: (+) and (−)-2-amino-1-butanol, dehydroabietylamine, (+) and (−)-α-4-bromophenylethylamine, (−)-α-(1-naphthyl)ethylamine, *l*-ephedrine, 1*S*-2*S*-(+)-2-amino-1-phenyl-1,3-propanediol, (+) and (−)-2-(4-chlorobenzylamino)-1-butanol, (+) and (−)-2-(4-thiobenzylamino)-1-butanol, (+) and (−)-2-(3-nitrobenzylamino)-1-butanol, and (+) and (−)-2-(2,5-dimethylbenzylamino)-1-butanol.

Preferably, the racemic acid of formula (III) is dissolved in a toluene/water mixture having a weight ratio of about 40/60 to 60/40, and preferably having a weight ratio of 50/50 to 55/45.

To the above two-phase mixture is then added (−)-α-phenethylamine in amounts from about 50 to 70 mol percent (preferably 60 to 70 mol percent), and sodium hydroxide in amounts from about 30 to 50 mol percent, wherein the sum of these two components is preferably 100 mol percent. The reaction mixture is then stirred and heated at a temperature ranging from about 40 to 100°C (preferably from about 70 to 80°C) for a period of time sufficient to complete the formation of the (−)-α-phenethylamine salt of (+)-2-(*p*-difluoromethoxyphenyl)-3-methylbutyric acid. The reaction mixture is then cooled and the aqueous phase is separated from the toluene phase which contains the above salt. The salt may be isolated from the organic phase by conventional methods (i.e., filtration) and used without further purification, or it may be purified by standard methods (i.e., recrystallization) if so desired.

Finally, the salt is decomposed with dilute sulfuric acid, or dilute hydrochloric acid, in the presence of toluene and water to yield the desired (+)-2-(*p*-difluoromethoxyphenyl)-3-methylbutyric acid of formula (VII) dissolved in the toluene phase. The solution is dried and the acid isolated therefrom, or the solution may be used directly in the preparation of the pyrethroid. The reaction for the preparation of the pyrethroid is the same as described above, excepting that the (+) acid of formula (VII) is substituted for the racemic acid of formula (III).

To the solution of the (+) formula (VII) acid in toluene is added thionyl chloride in amounts in excess over theory. The mixture is then heated at reflux until essentially all of the carboxylic acid of formula (VII) is converted to the corresponding acid chloride of formula (IVa). Next, the excess thionyl chloride is removed from the reaction mixture, and the acid chloride isolated by removal of the solvent under vacuum; or, if so desired, the solution of said acid chloride may be used directly. Finally, the acid chloride is reacted with (±)-*m*-phenoxybenzyl alcohol or (±)-α-cyano-*m*-phenoxybenzyl alcohol formula (Va) in the presence of toluene and pyridine to yield the pyrethroid of formula (VIa) shown below.

As stated above, the thus-obtained pyrethroid is characterized by a pesticidal activity increased twofold over that of the analog prepared from the unresolved formula (III) acid.

The reaction may be graphically illustrated as follows:

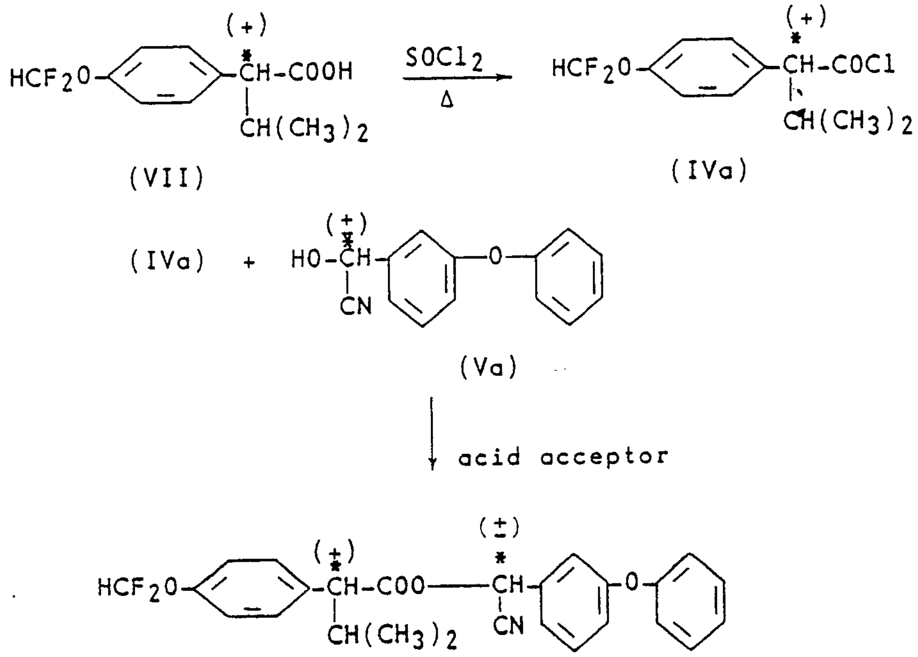


(VII)

(IVa)

(IVa) +

(Va)

(VIa)

The pesticide of formula (VIa) is a two isomer mixture, derived from the (+) acid and the (±) alcohol, as shown above; wherein the asterisks indicate the two chiral centers present in said pesticide.

Obviously, in the course of resolving the formula (III) racemic acid to obtain the desired (+) acid formula (VII) required in the above reaction sequence, a significant amount of (−) acid of formula (VIII) is also obtained. It is of interest, therefore, to recycle the above (−) acid *via* a racemization-resolution process in order to obtain additional amounts of the (+) isomer of said formula (III) acid.

Thus, it is desirable to provide a method for the preparation of (+)-2-(4-difluoromethoxyphenyl)-3-methylbutyric acid formula (VII) *via* the esterification, racemization, and hydrolysis of formula (VIII) (−) acid followed by the resolution of the thus-obtained racemic acid.

Conveniently, one molar equivalent of a $C_1$—$C_8$ alkyl ester, preferably methyl ester of a (−) enriched formula (VIII) acid is dissolved in an anhydrous $C_1$—$C_8$ alcohol, preferably methanol. Next, from about one to about two molar equivalents, and preferably 1.25 to 1.50 molar equivalents of sodium hydroxide or potassium hydroxide is added. The reaction mixture is then heated at a temperature from about 60 to about 100°C and preferably 68 to 72°C, or at the boiling point of the alcohol selected, for a period of time from about two to about ten hours and preferably six to seven hours or until the reaction is essentially complete. Next, if so desired, the solvent is removed, as under vacuum.

The residue is dissolved in water and the racemic acid mixture precipitated with a strong mineral acid such as hydrochloric acid. This reaction sequence is illustrated as follows:

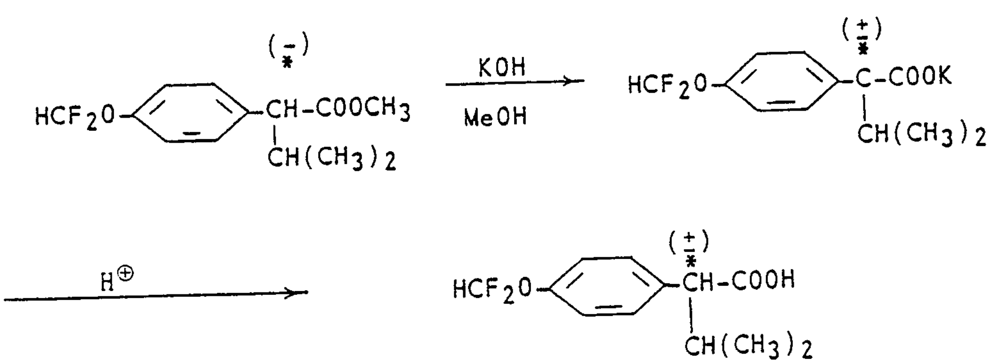


The thus-obtained racemic (±) acid of formula (III) is then recycled for admixture with the formula (III) racemic acid and resolved as described above.

The above-described process involves freonation of a *p*-substituted phenol to yield a formula (I)

difluoromethoxyaromatic compound. It likewise provides the option for further processing of said difluoromethoxyaromatic compound, when R in said formula (I) is

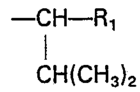

and $R_1$ is CN or $COOR_2$ and $R_2$ is $C_1$—$C_8$ alkyl. The further processing steps include: hydrolyzation, resolution, esterification, racemization, and recyclization. The overall process is graphically illustrated in Flow Diagram II below.

The invention is further described and illustrated by the examples set forth below.

FLOW DIAGRAM II

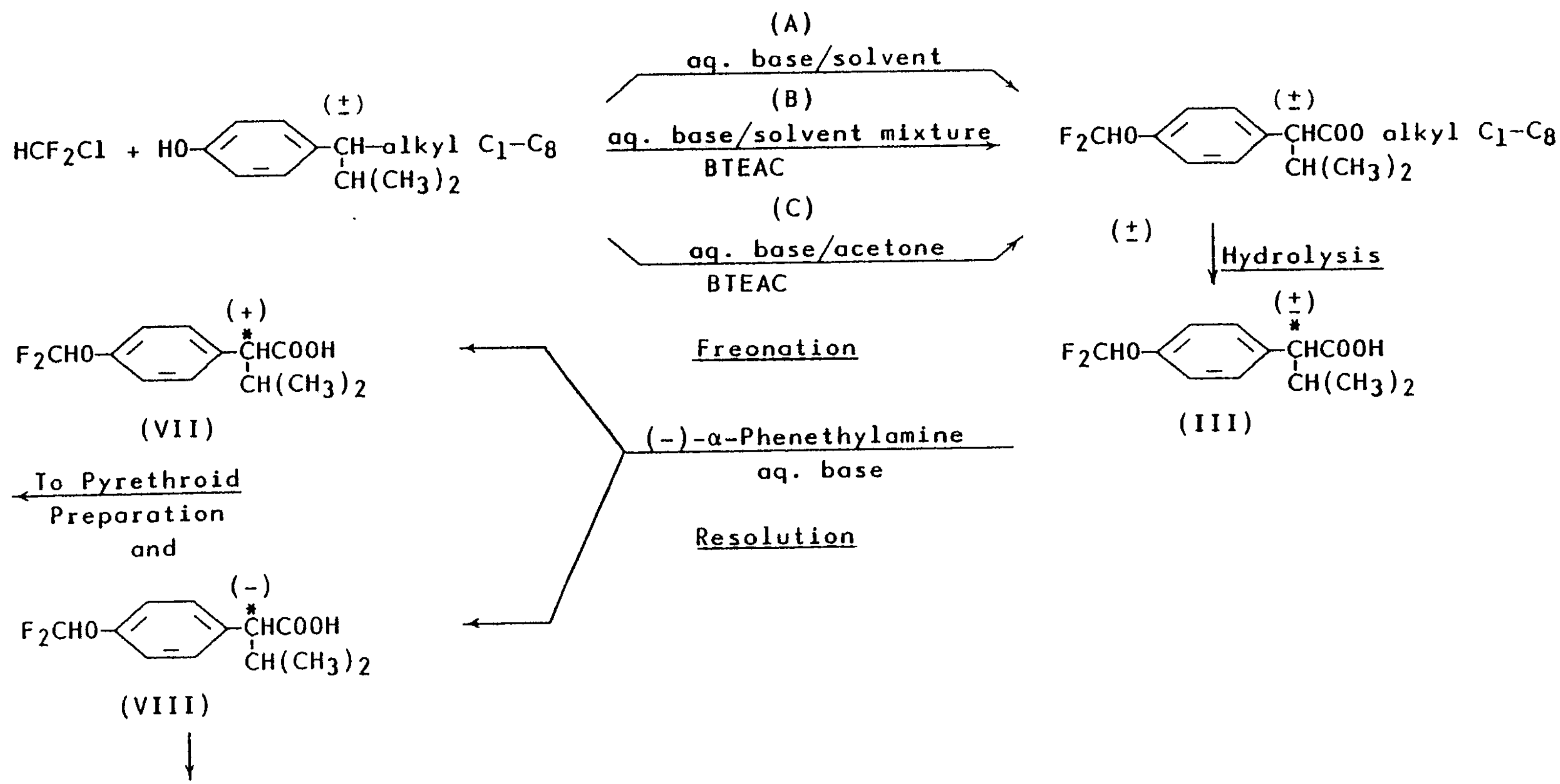


(Continued on next page)

FLOW DIAGRAM II (Continued)

(VIII) (-) acid

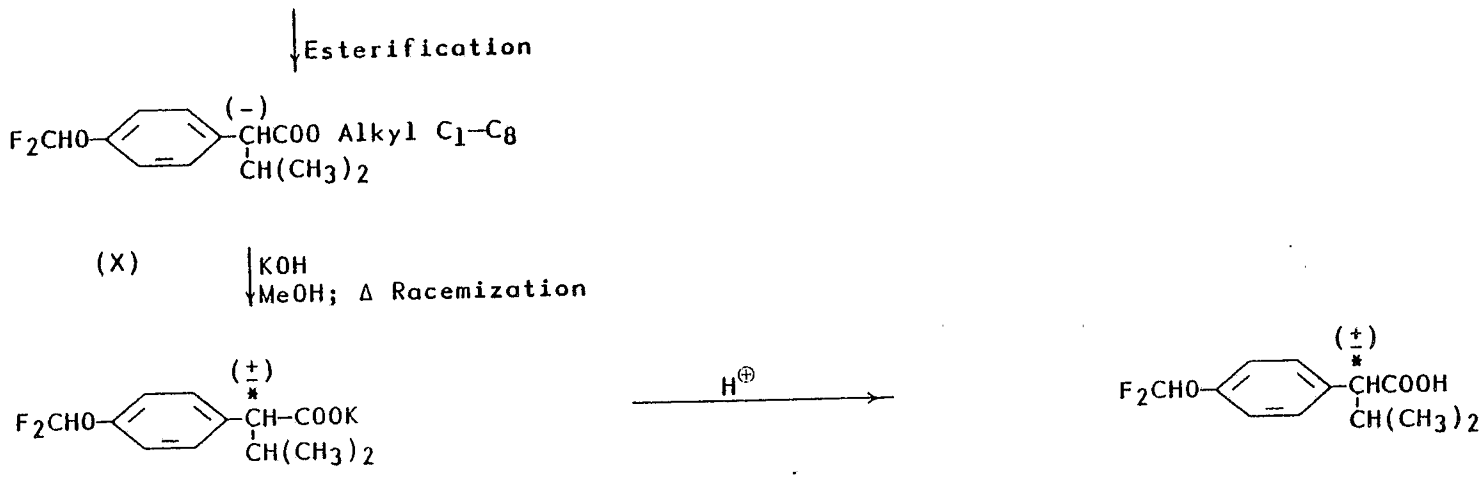


(X)

(III)

Recycle

Example 1

Preparation of Methyl 2-[4-(difluoromethoxy)phenyl]-3-methylbutyrate

A mixture of methyl 2-(4-hydroxyphenyl)-3-methylbutyrate (44.42 g of 93.8% pure material = 41.67 g; 0.20 mol), acetone (83.3 ml), 2-propanol (83.3 ml), and water (62.8 ml) is stirred at 30°C in a closed system. After the system is evacuated, chlorodifluoromethane is introduced into the reaction mixture under a pressure of 0.49 kg $cm^{-2}$. After about 5 minutes, one equivalent of 50.6% of aqueous sodium hydroxide (10.4 ml; 0.2 mol) from a pressurized reservoir is added all at once causing the reaction mixture to exotherm to 34°C with a concomitant rise in pressure to 0.7 kg $cm^{-2}$. Slow addition of the rest of 50.6% aqueous sodium hydroxide (31.6 ml; 0.60 mol) then commences and is completed in about 60 minutes. About 5 minutes after the beginning of the slow addition of the base, the pressure under which the chlorodifluoromethane is introduced into the reaction mixture is increased to 1.05 kg $cm^{-2}$. In about 45 minutes a total of 0.60 mol of chlorodifluoromethane (51.66 g) is added. After the addition of the sodium hydroxide is completed, the reaction mixture is stirred for an additional hour. The system is then evacuated to remove any unreacted chlorodifluoromethane from the reaction mixture. Next, a 1:1 mixture (by volume) of acetone:2-propanol (100 ml) is added while washing down the reactor. The reaction mixture is filtered and the organic layer is separated yielding 334.6 g of an orange liquid.

An aliquot (139.44 g) 41.67% of the total) of the above liquid is evaporated under vacuum to remove the solvents. The residue is diluted with toluene (100 ml), and the toluene solution is first washed with 5% aqueous sodium hydroxide (2 × 75 ml) and then with water (75 ml). It is then evaporated under vacuum to yield 21.26 g of a clear, reddish-orange liquid (calculated yield:

$$\frac{21.26}{41.67} \times 100 = 51.02 \text{ g}$$

or 98.9%). Analysis (glc) indicates this sample to be 86.5% pure corresponding to a real yield of 85.4%.

Another sample prepared by the above method is further purified by vacuum distillation; bp. 58—59°C. at 0.025 mm Hg.

*Analysis*

Calculated for $C_{13}H_{16}F_2O_3$: C 60.45; H 6.25; F 14.71; found: C 60.87; H 6.45; F 18.01.

By the above process, a number of experiments are run to evaluate the effect of various solvents, and combinations thereof, alone or in conjunction with other variables on the yields of the product obtained by said process.

In these reactions, the purity of the methyl 2(4-hydroxyphenyl)-3-methylbutyrate is in the range of from about 85% to about 95%. The reactions are run on a 0.2 to 0.3 mole scale at a temperature range of from about 30 to about 35°C, at a pressure range of from 0.49 kg $cm^{-2}$ to 2.45 kg $cm^{-2}$. Under these conditions the chlorodifluoromethane reactant is added over a time period ranging from about 30 mintues to 5.0 hours (in excess amounts as indicated). Following the addition of the aqueous base, the reaction mixtures are held for a time period ranging from 0 to 4 hours (usually 1 hour) under the above-specified conditions prior to workup.

Data obtained as indicated above are summarized in Tables Ia, Ib and Ic.

TABLE Ia

Preparation of Methyl 2-[4-(difluoromethoxy)phenyl]-3-methylbutyrate

| No. | Mol % Water added at | | ml of solvent/g of a compound of formula II | | $ClCHF_2$ mol equivalent | Product | | |
|---|---|---|---|---|---|---|---|---|
| | start | end | 2-ProH* | 2nd solvent | | % crude | % purity | % yield |
| 1 | 871 | 1740 | 3 | $CH_3CN$ 3 | 3.0 | 99.24 | 81.9 | 81.3 |
| 2 | 871 | 1740 | 2 | $CH_3CN$ 2 | 3.0 | 97.81 | 82.5 | 80.7 |
| 3 | 1740 | 2611 | 2 | $CH_3CN$ 2 | 3.0 | 97.01 | 84.3 | 81.8 |
| 4 | 1740 | 2611 | 2 | acetone 2 | 3.0 | 95.16 | 84.7 | 80.6 |
| 5 | 1740 | 2611 | 2 | acetone 2 | 3.0 | 98.29 | 83.9 | 82.5 |
| 6 | 1740 | 2610 | 2 | acetone 2 | 2.7 | 95.43 | 84.3 | 80.4 |
| 7 | 1740 | 2611 | 2 | acetone 2 | 3.0 | 103.0 | 77.5 | 80.1 |
| 8 | 1740 | 2611 | 1 | acetone 3 | 3.0 | 96.32 | 83.3 | 80.2 |
| 9 | 1740 | 2611 | 2 | acetone 2 | 3.0 | 98.78 | 86.5 | 85.4 |
| 10 | 2614** | 3482 | 2 | acetone 2 | 3.0 | 83.04 | 86.1 | 71.5 |

* 2-ProH = 2-propanol
** reference

TABLE Ib (Reference)

Preparation of Methyl 2-[4-(difluoromethoxy)phenyl]-3-methylbutyrate

| No. | mol % water added at | | ml of solvent/g of a cpd. of formula II | $ClCHF_2$ mol equivalent | Product | | | Remarks |
|---|---|---|---|---|---|---|---|---|
| | start | end | 2-ProH** | | % crude | % purity | % yield | |
| 1 | 871 | 1740 | 6 | 3.0 | 88.48 | 78.0 | 69.0 | |
| 2 | 871 | 1740 | 6 | 3.0 | 87.00 | 82.2 | 71.5 | |
| 3 | 871 | 1740 | 6 | 3.0 | 98.04 | 78.0 | 76.5 | under 2.45 kg $cm^{-2}$ pressure |
| 4 | 871 | 1740 | 6 | 3.0 | 103.8 | 73.0 | 75.8 | under 2.45 kg $cm^{-2}$ pressure |
| 5 | 871 | 1740 | 6 | 3.0 | 95.21 | 78.3 | 74.5 | under 2.45 kg $cm^{-2}$ pressure |
| 6 | 871 | 1740 | 4 | 2.6 | 80.67 | 86.4 | 69.7 | |
| 7 | 871 | 1740 | 4 | 2.6 | 87.82 | 86.3 | 75.8 | |
| 8 | 871 | 1740 | 6 | 3.0 | 89.02 | 80.5 | 71.7 | |
| 9 | 1740 | 1740 | 6 | 2.9 | 82.88 | 81.2 | 67.3 | $ClCHF_2$ added last after all the base was added |
| 10 | 871 | 1740 | 6 | 3.0 | 79.98 | 84.1 | 67.3 | 10% by wt of toluene present* |

TABLE Ib (Reference) (continued)

Preparation of Methyl 2-[4-(difluoromethoxy)phenyl]-3-methylbutyrate

| No. | mol % water added at | | ml of solvent/g of a cpd. of formula II | $ClCHF_2$ mol equivalent | Product | | | Remarks |
|---|---|---|---|---|---|---|---|---|
| | start | end | 2-ProH** | | % crude | % purity | % yield | |
| 11 | 871 | 1740 | 6 | 3.0 | 80.07 | 84.2 | 67.4 | 10% by weight of toluene present* |
| 12 | 871 | 1740 | 6 | 3.0 | 82.73 | 84.0 | 69.5 | 10% by weight of toluene present* |
| 13 | 871 | 1740 | 6 | 3.0 | 83.93 | 83.7 | 70.2 | 10% by weight of toluene present* |
| 14 | 871 | 1740 | 6 | 3.0 | 81.60 | 85.9 | 70.1 | 10% by weight of toluene present* |
| 15 | 871 | 1740 | 6 | 3.0 | 91.90 | 80.7 | 74.2 | 5% by wt of xylene present* |

* = In these preparations the reaction mixture contained the amounts of solvent indicated, simulating the use of solutions of starting material, as isolated from preparative reactions thereof.

** 2-ProH = 2-propanol.

TABLE Ic (Reference)

Preparation of Methyl 2-[4-(difluoromethoxy)phenyl]-3-methylbutyrate

| No. | mol % water added at | | ml of solvent/g of a cpd. of formula II | | $ClCHF_2$ mol equivalent | Product | | |
|---|---|---|---|---|---|---|---|---|
| | start | end | acetone | $CH_3CN$ | | % crude | % purity | % yield |
| 1 | 1740 | 2610 | 2 | — | 2.3 | 74.32 | 85.5 | 63.5 |
| 2 | 1740 | 2611 | 3 | — | 3.0 | 85.44 | 84.4 | 72.1 |
| 3 | 2614 | 3482 | 4 | — | 3.0 | 73.28 | 85.7 | 62.8* |
| 4 | 1740 | 2611 | 4 | — | 3.0 | 93.16 | 82.5 | 76.9 |
| 5 | 2614 | 3482 | 4 | — | 3.0 | 84.26 | 85.5 | 72.0 |
| 6 | 2614 | 3482 | 4 | — | 3.0 | 82.20 | 85.7 | 70.4** |
| 7 | 871 | 1740 | 6 | — | 3.0 | 97.15 | 81.1 | 78.8 |
| 8 | 871 | 1740 | — | 4 | 3.0 | 97.01 | 80.3 | 77.9 |
| 9 | 871 | 1740 | — | 6 | 3.0 | 95.37 | 78.2 | 74.6 |

* hold time = 0 hr. ** hold time = 5 hr.
Hold time = time of stirring after base addition

It can be seen from the above Tables that product yields are improved when mixed solvents (2-propanol:acetone or 2-propanol:acetonitrile) are used in a 1:1 to 1:3 ratio preferably 1:1 ratio, wherein the combined volume of the solvents is in the range of from 4 to 6 ml per gram of starting material. Product yields are lower when the above solvents are used singularly and in amounts from 2 to 6 ml per gram of starting material.

## Example 2

Preparation of 2-[4-(difluoromethoxy)phenyl]-3-methylbutyronitrile

A mixture of 2-(4-hydroxyphenyl)-3-methylbutyronitrile (25.0 g; 0.1427 mol), 2-propanol (230 ml), and 25.8% aqueous sodium hydroxide (103 ml; 0.857 mol) is stirred in a pressure vessel and chlorodifluoromethane (36.4 g; 0.421 mol) is added over a period of 52 minutes with cooling to control the exotherm and to maintain the temperature of the reaction mixture below 52°C. The reaction mixture is then stirred for an additional 1½ hours. Water (700 ml) and ether (300 ml) are then added to the reaction mixture and the two phase mixture is separated. The ether solution is washed twice with water (1000 ml + 500 ml) and then with saturated brine (300 ml). It is then dried and evaporated under vacuum to yield 30.48 g (94.8%) of a clear, very slightly yellow liquid, shown to be 85.9% pure by gas-liquid chromatography. This corresponds to a yield of 81.4%.

The product is further purified by vacuum distillation, b.p. 106—109°C at 0.03 mm Hg.

*Analysis*

Calculated for $C_{12}H_{13}F_2NO$: C 63.99; H 5.89; N 6.22; F 16.87; found: C 64.02; H 5.90; N 6.38; F 16.95.

By the above procedure, but substituting ethyl 2-(4-hydroxyphenyl)-3-methylbutyrate for 2-(4-hydroxyphenyl)-3-methylbutyronitrile, 2-[4-(difluoromethoxy)phenyl]-3-methylbutyric acid ethyl ester is prepared, respectively. The product is purified by vacuum distillation, b.p. 84.0—85.5°C at 0.09 mm Hg.

*Analysis*

Calculated for $C_{14}H_{18}F_2O_3$: C 61.75; H 6.66; F 13.96; found: C 61.73; H 6.51; F 13.96.

## Example 3

Preparation of 4-chloro-α,α-difluoroanisole

A mixture of 4-chlorophenyl (25.71 g; 0.2 mol), acetone (83.3 ml), 2-propanol (83.3 ml), and water (62.8 ml) is stirred at 30°C. in a closed system. After the system is evacuated, chlorodifluoromethane is introduced into the reaction mixture under a pressure of 0.49 kg $cm^{-2}$. After about 5 minutes, 50.6% aqueous sodium hydroxide (10.4 ml; 0.2 mol) is added at once, followed by the dropwise addition of 50.6% aqueous sodium hydroxide at a rate of aproximately 2.6 ml/min, over a period of 59 minutes (total: 31.6 ml; 0.6 mol). Five minutes after the dropwise addition of the base commences, the pressure under which the chlorodifluoromethane is added, is increased to 1.05 kg $cm^{-2}$. In about 55 minutes the addition of chlorodifluoromethane is stopped (a total of 0.6 mol was added). After the addition of the sodium hydroxide, the reaction mixture is stirred for an additional hour.

Next, the system is evacuated to remove unreacted chlorodifluoromethane and about 150 ml of a 1:1 (by volume) mixture of acetone: 2-propanol is added as washes of the reactor to the reaction mixture, and the whole is then filtered. After filtration, the organic layer is separated from the aqueous layer and evaporated under vacuum to yield a light orange oil containing some solids. Hexane (200 ml) and water (150 ml) are added to the oil. The hexane layer is washed with 5% aqueous sodium hydroxide (150 ml), dried and evaporated under vacuum to yield 24.63 g (69%) of a clear, yellow oil. The oil is vacuum distilled to yield the product, b.p. 61.5—63.0°C. at 15—25 mm Hg.

*Analysis*

Calculated for $C_7H_5ClF_2O$: C 47.08; H 2.82; Cl 19.86; F 21.28; found: C 47.08; H 2.85; Cl 20.05; F 21.05.

By the above procedure, but substituting *p*-cresol for 4-chlorophenol,α,α-difluoro-4-methylanisole is obtained respectively; b.p. 52.5—53.5°C. at 15—25 mm Hg.

## Example 4

Preparation of α,α-difluoro-4-nitroanisole

The procedure of Example 3 is used, except that 4-nitrophenol is substituted for 4-chlorophenol.

The organic layer is evaporated under vacuum. The residue obtained is taken up in a mixture of hexane (150 ml) and water (150 ml) but it is only partially soluble in same. The mixture is filtered to yield 28.2 g (74%) of a slightly yellow solid. On evaporation, the hexane layer yields 3.88 g (10.3%) of a solid. The fractions are combined and recrystallized from a hexane-methylene chloride mixture, mp 32.5—35.5°C. [reported mp 32—32.5°C.; T. G. Miller and J. W. Thanassi, J. Org. Chem. *25*: p. 2009 (1960)].

## Example 5

Preparation of Methyl 2-[4-(difluoromethoxy)phenyl]-3-methylbutyrate

A mixture of methyl 2-(4-hydroxyphenyl)-3-methylbutyrate (0.2 to 0.3 mol), a solvent of 1:1 acetone:2-propanol mixture (used at the rate of 4 ml/g of the above compound), benzyltriethylammonium chloride

(BTEAC; 0 to 10 mol percent) and water (1740 to 2614 mol percent) is stirred at 30 to 35°C in a closed system. The system is evacuated and chlorodifluoromethane (3 molar equivalents) is introduced into the reaction mixture under a pressure of from 0.45 to 1.1 kg $cm^{-2}$ over a period of about 30 minutes to 1.0 hour. About 5 minutes after the start of the chlorodifluoromethane addition, one molar equivalent of 50.6% aqueous sodium hydroxide is added all at once (causing a slight exotherm). The slow addition of three molar equivalents of 50.6% aqueous sodium hydroxide commences and is completed in about the same period of time needed to add the chlorodifluoromethane. On completion of the additions the reaction mixture is stirred an additional 0 to 1 hour. The system is then evacuated to remove any unreacted chlorodifluoromethane from the reaction mixture. The organic layer is separated and evaporated under vacuum. The residue is dissolved in toluene. The toluene solution is washed with dilute aqueous sodium hydroxide and then with water. The solvent is then evaporated under vacuum to yield an orange liquid, the title product.

Several experiments are run by the above procedure, and the data thus obtained are summarized in Table II below. It can be clearly seen from Table II that the combination of 1:1 acetone:2-propanol + 10 mol percent BTEAC affords the highest yields attained by utilizing the above procedure.

By the above procedure, but substituting ethyl 2-(4-hydroxyphenyl)-3-methylbutyrate, 2-(4-hydroxyphenyl)-3-methylbutyronitrile, 4-chlorophenol, *p*-cresol or 4-nitrophenol for methyl 2-(4-hydroxyphenyl)-3-methylbutyrate, 2-[4-(difluoromethoxy)phenyl]-3-methylbutyric acid ethyl ester, 4-chloro-α,α-difluoroanisole, α,α-difluoro-4-methylanisole or α,α-difluoro-4-nitroanisole can be prepared, respectively.

TABLE II (Reference)

Preparation of Methyl 2-[4-(difluoromethoxy)phenyl]-3-methylbutyrate in the presence of BTEAC

| No. | Mole % water added at | | ml of solvent per g of formula II | | BTEAC* mol percent | Product | | |
|---|---|---|---|---|---|---|---|---|
| | start | end | 2-ProH** | acetone | | % crude | % purity | % yield |
| 1 | 2614 | 3482 | 2 | 2 | — | 83.04 | 86.1 | 71.5 |
| 2 | 2614 | 3482 | 2 | 2 | 2.5 | 83.87 | 86.4 | 72.5 |
| 3 | 2614 | 3482 | 2 | 2 | 10 | 92.21 | 85.7 | 79.0 |

* BTEAC = Benzyltriethylammonium chloride
** 2-ProH = 2-propanol

Example 6

Preparation of Methyl 2-[4-(difluoromethoxy)phenyl]-3-methyl butyrate

A mixture of methyl 2-(4-hydroxyphenyl)-3-methylbutyrate (0.2 to 0.3 mol) a solvent selected from acetone or a 1:1 acetone: 2-propanol mixture (used at the rate of 4 ml/g of the above compound), benzyltriethylammonium chloride (BTEAC; 0 to 10 mol percent) and water (1740 to 2614 mol percent) is stirred at 30 to 35°C in a closed system. The system is evacuated and chlorodifluoromethane (3 molar equivalents) is introduced into the reaction mixture under a pressure of from 0.45 to 1.1 kg $cm^{-2}$ over a period of about 30 minutes to 1.0 hour. About 5 minutes after the start of the chlorodifluoromethane addition, one molar equivalent of 50.6% aqueous sodium hydroxide is added all at once (causing a slight exotherm). The slow addition of three molar equivalents of 50.6% aqueous sodium hydroxide begins and is completed in about the same period of time needed to add the chlorodifluoromethane. On completion of the additions, the reaction mixture is stirred an additional 0 to 1 hour. Next, the system is evacuated to remove any unreacted chlorodifluoromethane from the reaction mixture. The organic layer is separated and is then evaporated under vacuum. The residue is dissolved in toluene. The toluene solution is washed with dilute aqueous sodium hydroxide and then with water. The solvent is then evaporated under vacuum to yield an orange liquid, the title product.

Several experiments are run by the above procedure, and the data thus obtained are summarized in Table III below. It can be clearly seen from Table III that the combination of acetone + 10 mol percent of BTEAC affords the highest yields attained by utilizing the above procedure.

By the above procedure, but substituting ethyl 2-(4-hydroxyphenyl)-3-methylbutyrate, 2-(4-hydroxyphenyl)-3-methylbutyronitrile, 4-chlorophenol, *p*-cresol or 4- nitrophenol for methyl 2-(4-hydroxyphenyl)-3-methylbutyrate, 2-[4-(difluoromethoxy)phenyl]-3-methylbutyric acid ethyl ester, 4-chloro-α,α-difluoroamisole, α,α-difluoro-4-methylamisole or α,α-4-nitroamisole can be prepared, respectively.

TABLE III

Preparation of Methyl 2-[4-(difluoromethoxy)phenyl]-3-methylbutyrate in the presence of BTEAC

| No. | Mol % water added at | | ml of acetone per g of formula (II) | BTEAC* mol percent | Product | | |
|---|---|---|---|---|---|---|---|
| | start | end | | | % crude | % purity | % yield |
| 1 | 1740 | 2611 | 4 | — | 93.16 | 82.5 | 76.9 |
| 2** | 2614 | 3482 | 4 | — | 84.26 | 85.5 | 72.0 |
| 3** | 2614 | 3482 | 4 | — | 73.28 | 85.7 | 62.8 |
| 4 | 1740 | 2611 | 4 | 10 | 97.40 | 82.8 | 80.6 |
| 5** | 2614 | 3482 | 4 | 10 | 97.13 | 82.3 | 79.9 |
| 6** | 2014 | 3482 | 4 | 10 | 96.43 | 83.4 | 80.4 |

* BTEAC = Benzyltriethylammonium chloride
** Reference

Example 7

Preparation of the (−)-α-phenethylamine salt of (+)-2-(*p*-difluoromethoxyphenyl)-3-methylbutyric acid

A solution of (+)-2-(*p*-difluoromethoxyphenyl)-3-methylbutyric acid in toluene (9.0 kg as is, 3.75 kg crude acid = 3.1 kg pure; 12.5 mol), water (6.75 l), and toluene (3.75 l), and 50% sodium hydroxide (608 g = 398 ml = 7.6 mol) is stirred, heated to 84 to 86°C and a solution of (−)-α-phenethylamine (PEA: 921 g, 7.6 mol) in toluene (9.57 l) is added over a 10 to 15 minute period. Shortly after the addition is completed, the title product precipitates. The reaction mixture is held for one hour at reflux and is then cooled to 40 to 45°C and allowed to settle. Next, the aqueous phase is removed, the toluene layer (a slurry) is washed with 10% sodium chloride, or sodium sulfate solution (5.0 kg), the aqueous wash is removed and toluene (6.25 l) is added to the organic phase. The mixture is heated to reflux and held at reflux for 1.5 hours. It is then cooled to 40°C and filtered. The filter cake is washed with water (6.5 l) and toluene (6.5 l), and is then used without drying in the next reaction step.

Example 8

Preparation of (+)-2-(*p*-difluoromethoxyphenyl)-3-methylbutyric acid

The wet filter cake obtained by the preparation of Example 1 is added slowly to a well stirred mixture of toluene (1.75 l) and 20% aqueous sulfuric acid (2.7 kg; 2.37 l) at below 45°C. After stirring the mixture for a short period of time, the layers are allowed to settle (the pH of the aqueous layer must be $<2$). The aqueous layer is then separated and the toluene layer containing the title product may be used without further purification in the reaction yielding the pyrethroid insecticide.

In the above reaction, the title product is obtained in 80% yield as calculated from optical rotation data.

Example 9

Evaluation of the effect of various PEA/NaOH ratios on the optical purity and yield of (+)-2-(*p*-difluoromethoxyphenyl)-3-methylbutyric acid

By the method of Examples 7 and 8, a number of preparations are run in toluene/water media using various mol percent ratios of (−) α-phenethylamine (PEA) and sodium hydroxide. Pertinent data and the results of these preparations are summarized in Table IV below.

It can be clearly seen from Table IV that the best yields of highly pure (optically) products are obtained when the amount of PEA is from about 50 to 70 mol percent and the sum of PEA and sodium hydroxide adds up to at least 100 mol percent.

TABLE IV

Evaluation of the effect of variations in the mol % of PEA and NaOH on the optical purity and yield of (+)-2-(*p*-difluoromethoxyphenyl)-3-methylbutyric acid

| No. | Toluene/water ratio | mol % PEA | mol % NaOH | % crude product$^+$ | $[\alpha]_D^{RT}$ $CHCl_3$ in degrees | % (+)-acid present in crude product$^{++}$ | % yield of (+)-acid$^+$ |
|---|---|---|---|---|---|---|---|
| 1 | 100/0 | 45 | — | 67.3** | 36.9 | 92.0 | 61.9** |
| 2 | 55/45 | 40 | 50 | 71.6 | 39.1 | 97.3 | 69.7 |
| 3 | 50/50 | 45 | 55 | 88.2** | 33.0 | 87.5 | 77.2** |
| 4 | 55/45 | 50 | 40 | 76.1 | 31.3 | 87.8 | 66.8 |
| 5 | 55/45 | 50 | 45 | 83.6 | 38.1 | 96.0 | 80.3 |
| 6 | 55/45 | 50 | 50 | 87.3* | 36.8 | 95.6* | 83.5* |
| 7 | 50/50 | 50 | 50 | 94.7** | 32.5 | 87.0 | 82.4** |
| 8 | 55/45 | 50 | 60 | 85.4* | 36.1 | 97.7* | 83.4* |

TABLE IV (continued)

Evaluation of the effect of variations in the mol % of PEA and NaOH on the optical purity and yield of (+)-2-(*p*-difluoromethoxyphenyl)-3-methylbutyric acid

| No. | Toluene/water ratio | mol % PEA | mol % NaOH | % crude product+ | $[\alpha]_D^{RT}$ $CHCl_3$ in degrees | % (+)-acid present in crude product++ | % yield of (+)-acid+ |
|---|---|---|---|---|---|---|---|
| 9 | 55/45 | 60 | 50 | 94.8* | 37.0 | 95.8* | 90.8* |
| 10 | 55/45 | 60 | 50 | 98.2* | 37.4 | 95.9* | 94.2* |
| 11 | 55/45 | 60 | 55 | 96.3* | 38.2 | 95.2* | 91.7* |
| 12 | 55/45 | 60 | 55 | 92.7 | 36.0 | 93.5 | 86.7 |
| 13 | 55/45 | 65 | 55 | 98.0 | 35.8 | 93.0 | 91.7 |
| 14 | 55/45 | 70 | 55 | 95.1 | 33.8 | 90.8 | 86.4 |
| 15 | 50/50 | 70 | 30 | 131.6** | 11.7 | 63.4 | 83.4** |

\+ = purity of racemic acid included in calculations, excepting data marked with: **
++ = calculated from optical rotation data
* = chemical purity of (+) acid was included in these calculations

Example 10

Comparison of the use of toluene and xylene in the resolution of the racemic mixture and the results obtained thereby

By the methods of Examples 7 and 8, excepting that 4.1 kg of racemic acid is used in each test, the use of toluene and xylene on the resolution of racemic acid is compared. The results obtained are shown below.

| | Toluene | Xylene |
|---|---|---|
| % yield of as is (+)-acid | 83.9 | 85.5 |
| $[\alpha]_D^{RT}$ methanol, in degrees | 36.8 | 34.6 |
| % (+)-acid | 91.9 | 89.4 |
| chemical purity of (+)-acid (%) | 97.7 | 98.6 |
| % real yield | 77.1 | 76.4 |

Comparable results are obtained using benzene as a solvent.

Example 11

One-step racemization and hydrolysis of the methyl ester of (−)-enriched-2-(4-difluoromethoxyphenyl)-3-methylbutyric acid

To a solution of 243.0 g {0.941 mol; $[\alpha]_D$ = −21.22°; ($CHCl_3$, c = 12.753 g/100 mL)} of methyl 2-(4-difluoromethoxyphenyl)-3-methylbutyrate in 972 mL of anhydrous methanol is added slowly 77.6 g (65.98 g real) of potassium hydroxide pellets (1.176 mol, or 125 mol %). The reaction mixture is refluxed at 68—70°C for six hours and allowed to cool slowly to room temperature. The solvent is then removed under vacuum, the residue washed with 400 mL cold water and extracted twice (400 mL and 200 mL) with hexane. The aqueous phase is acidified with concentrated hydrochloric acid, using ice to keep the mixture cool. The acidified mixture is extracted twice (2 × 400 mL) with hexane. The hexane solution is washed with water, saturated brine, dried over sodium sulfate and evaporated to afford 185.2 g of an oil which solidifies on standing {80.7%; $[\alpha]_D$ = −1.40 ($CHCl_3$, c = 1.059 g 100 mL)}. The product contains approximately 51.6% of (−)- and 48.4% of (+)-2-(4-difluoromethoxyphenyl-3-methylbutyric acid.

Example 12

Preparation of the methyl ester of (−)-enriched-2-(4-difluromethoxyphenyl)-3-methylbutyric acid

A solution of 250 g {1.023 mol; $[(\alpha]_D$ = −21.04° ($CHClo_3$, c = 2.385 g/100 mL)} of (−)-enriched-2-(4-difluoromethoxyphenyl)-3-methylbutyric acid in 1000 mL of toluene and 0.5 mL DMF is heated at 70°C and 121.7 g (1.023 mol; 74.7 mL) of thionyl chloride is added over 30 minutes at such a rate as to provide control over the evolution of gases. After the addition is completed, the reaction mixture is heated at 100°C.

After one hour, the mixture is cooled to 15—20°C and 79.8 g (82.3 mL) pyridine (1.023 mol) added. The reaction mixture is stirred and 39.3 g (49.8 mL; 1.228 mol; or 125 mol %) of anhydrous methanol added at a rate to maintain the temperature of the mixture below 40—45°C. After the addition is completed, the reaction mixture is stirred for 15 minutes, cooled and diluted with water. The organic phase is separated and washed in succession with 400 mL of 10% hydrochloric acid, 400 mL of 5% sodium hydroxide and twice with 400 mL water each. The solution is then dried over sodium sulfate and evaporated to afford 243.7 g of a brown oil, the product; $[\alpha]_D$ = 21.22° ($CHCl_3$, c = 1.753 g/100 mL).

Example 13

Preparation of α-cyano-3-phenoxybenzyl-2-(4-difluoromethoxyphenyl)-3-methylbutyrate

A solution of 2-(4-difluoromethoxyphenyl)-3-methylbutyryl chloride (4.58 mmol; prepared by the method of Example 12 in either (5 mL) is added to an ether (20 mL) solution of α-cyano-3-phenoxybenzyl alcohol (4.58 mmol) and pyridine (0.5 mL). The mixture is stirred overnight and filtered. Filtrate and washing are evaporated and the residual oil is purified on 5 × 2 mm silica gel plates using 1:1 methylene chloride:hexane eluent to afford the title product.

The insecticidal activity of the title compound, Example 14, is reported in United States Patent No. 4,199,595, issued April 22, 1980.

**Claims**

1. A method for the preparation of a compound of the structural formula:

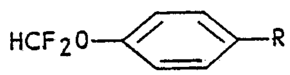

(I)

wherein R is $C_1$—$C_3$ alkyl, halogen, nitro, or R is the moiety

—CH—$R_1$
|
$CH(CH_3)_2$

wherein $R_1$ is —CN, —$COOR_2$, OH or $OR_3$, and $R_2$ is $C_1$—$C_8$ alkyl, and $R_3$ is tosyl, mesyl, or $C_2$—$C_4$ alkanoyl, by alkylating a *p*-substituted phenol having the formula:

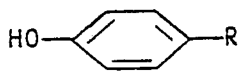

(II)

wherein R is as described above, with excess chlorodifluoromethane at atmospheric or superatmospheric pressure in the presence of a base, water and an inert water miscible organic solvent or solvent mixture, alone or in the presence of benzyltriethylammonium chloride, to yield the formula (I) difluoromethoxyaromatic compound, characterized in that the solvent is a mixture of 2-propanol:acetonitrile or 2-propanol:acetone, in 1:1 to 1:3 volume ratios, and further characterized in that said solvent mixture is used in amounts of from 4 to 6 ml per gram of the compound of formula (II) in the initial presence of from approximately 870 to 1740 mol percent of water, with two to three molar equivalents of chlorodifluoromethane added.

2. A method according to Claim 1, characterized in that the reaction is carried out at a temperature of about 20 to 40°C.

3. A method according to one of Claims 1 or 2, characterized in that the reaction is carried out under a pressure of from 0.4 to 2.5 kg $cm^{-2}$ over a period of time ranging from approximately 30 minutes to three hours and with an aqueous solution of four molar equivalents of an alkali metal hydroxide of sodium or potassium hydroxide, wherein the concentration of said aqueous solution is such that on completion of the base addition, the total amount of water added in the reaction mixture is of from approximately 1740 to 2610 mol percent is added essentially simultaneously with the chlorodifluoromethane addition over a period of time ranging from approximately 1.0 to 5.0 hours; and on completion of the base addition, the reaction mixture is further maintained at the above pressure and temperature ranges for a period of time of from 0 to 6 hours, or until said reaction is essentially complete.

4. A method according to one of Claims 1 to 3, characterized in that the reaction is optionally followed by hydrolyzing the thus-prepared ester by heating the same with a strong base in anhydrous methanol, thereafter removing the solvent from the reaction mixture, dissolving the residue in water and treating the resulting solution with strong mineral acid to yield the 2-(*p*-difluoromethoxyphenyl)-3-methylbutyric acid and admixing one molar equivalent of racemic 2-(*p*-difluoromethoxyphenyl)-3-methylbutyric acid in the presence of a mixture of water and a water immiscible solvent having a range of ratios of from 40/60; to 60/40 by weight, with an optically active amine used in amounts sufficient to form a salt with the dextrorotatory component of said racemic acid, and an inorganic water soluble base used in amounts sufficient to total the sum of said base and of the above amine to at least 100 mol percent; heating the thus-obtained mixture at a temperature range of from 40°C to that of the boiling point of said mixture for a period of time sufficient to form the optically active amine salt of said dextrorotatory acid, and isolating said salt from the rest of the reaction mixture and decomposing same with a mineral acid used in amounts sufficient to regenerate said dextrorotatory acid therefrom.

5. A method according to one of Claims 1 to 3, characterized in that the reaction is optionally followed by hydrolyzing the thus-prepared ester by heating the same with a strong base in anhydrous methanol, thereafter removing the solvent from the reaction mixture, dissolving the residue therefrom in water and treating the thus-obtained solution with hydrochloric acid to yield the 2-(*p*-difluoromethoxyphenyl)-3-methylbutyric acid and admixing one molar equivalent of racemic 2-(*p*-difluoromethoxyphenyl)-3-methylbutyric acid in the presence of a mixture of water and a water immiscible solvent selected from benzene, toluene, and xylene and having a range of ratios of from 40/60 to 60/40 by weight, with an optically active amine selected from (−)-α-phenethylamine, (+) and (−)-2-amino-1-butanol, dehydroabiethylamine, (+) and (−)-α-4-bromophenylethylamine, (−)-α-(l-naphthyl)ethylamine, *l*-Ephedrine, 1*S*-2*S*-(+)-2-amino-1-phenyl-1,3-propanediol (+) and (−)-2-(4-chlorobenzylamino)-1-butanol, (+) and (−)-2-(4-thiobenzylamino)-1-butanol, (+) and (−)-2-(3-nitrobenzylamino)-1-butanol, or (+) and (−)-2-(2,5-dimethylbenzylamino)-1-butanol used in amounts of from 40 to 100 mol percent; used in amounts sufficient to form a salt with the

dextrorotatory component of said racemic acid, and an inorganic water soluble base selected from alkali metal hydroxides, carbonates, or ammonium hydroxide used in amounts sufficient to total the sum of said base and of the above amine to at least 100 mol percent; heating the thus-obtained mixture at a temperature range of from 40°C to that of the boiling point of said mixture for a period of time sufficient to form the optically active amine salt of said dextrorotatory acid, isolating said salt from the rest of the reaction mixture and decomposing said with a mineral acid used in amounts sufficient to regenerate said dextrorotatory acid therefrom; acidifying the remaining portion of the above-said reaction mixture containing the amine salt of the levorotatory (−) acid with a mineral acid to obtain the levorotatory (−) acid, esterifying said (−) acid using an appropriate alcohol and a catalytic amount of a strong mineral acid to yield the coresponding (−) ester of (−)-2-(4-difluoromethoxyphenyl)-3-methylbutyric acid, reacting one molar equivalent of said $C_1$—$C_8$ alkyl ester of (−)-2-(4-difluoromethoxyphenyl)-3-methylbutyric acid with one to two molar equivalents of sodium or potassium hydroxide in the presence of anhydrous $C_1$—$C_3$ alcohol at a temperature from 60 to 100°C or at the boiling point of the alcohol selected, for a period of time from two to ten hours or until the reaction is essentially complete and the racemic potassium salt of the 2-(4-difluoromethoxyphenyl)-3-methylbutyric acid is formed, separating the solvent from the reaction mixture, dissolving the remaining residue in water, acidifying the aqueous solution with strong mineral acid to obtain the racemic 2-(4-difluoromethoxyphenyl)-3-methylbutyric acid and recycling the same for resolution thereof.

6. A method according to Claim 1, wherein to a solution at a temperature of from 30 to 35°C. of one molar equivalents of a compound of formula -(II) in a solvent mixture of 2-propanol:acetone wherein the volume ratio of said solvent is 1:1 and in the total volume amount of 4 ml per gram of a compound of formula -(I) and water which is added initially at the rate of 1740 mole percent is added 3 molar equivalents of chlorodifluoromethane over a time period ranging from 30 minutes to 1.0 hour at a pressure of from approximately 0.4 kg $cm^{-2}$ to 1.1 kg $cm^{-2}$ along with initially the simultaneous addition of 4 molar equivalents of an aqueous sodium hydroxide solution which is added over a period of time of approximately 1.0 hour to 2.0 hours, and on the completion of the base addition, the total amount of water added is 2610 mol percent and the reaction is further maintained at the above pressure and temperature range for a period of time of from approximately 1 to 2 hours.

7. A method according to Claim 1, wherein 3 molar equivalents of chlorodifluoromethane are added over a 30 minute to 1.0 hour period at a pressure from about 0.4 to 1.1 kg $cm^{-2}$, at a temperature of from about 30 to 35°C, and further maintained at the above pressure and temperature ranges for about 1 to 2 hours; in the presence of 2614 mol percent water, 1:1 2-propanol:acetone used at a rate of 4 ml per gram of the compound of formula-(II); the alkali metal hydroxide is sodium hydroxide; and the total amount of water added to the reaction mixture is 3482 mol percent.

8. A method according to Claim 1, wherein 3 molar equivalents of chlorodifluoromethane are added over about a 30 minute to 1.0 hour period at a pressure from about 0.4 to 1.12 kg $cm^{-2}$, at a temperature from about 30 to 35°C, and further maintained at the above pressure and temperature ranges for about 1 to 2 hours; and the alkali metal hydroxide is sodium hydroxide.

9. A method according to any of Claims 4 to 6, wherein said compound is methyl 2-[4-(difluoromethoxy)phenyl]-3-methylbutyrate.

10. A method according to any of Claims 4 to 6, wherein said compound is ethyl 2-[4-(difluoromethoxy)phenyl]-3-methylbutyrate.

11. A method according to any of Claims 4 to 6, wherein said compound is 2-[4-(difluoromethoxy)phenyl]-3-methylbutyronitrile.

12. A method according to any of Claims 4 to 6, wherein said compound is 4-chloro-α,α-difluoroanisole.

**Patentansprüche**

1. Verfahren zur Herstellung einer Verbindung mit der Strukturformel

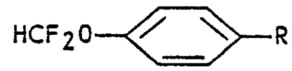

(I)

wobei R für $C_1$—$C_3$-Alkyl, Halogen, Nitro steht oder R für die Einheit

$$-CH(CH(CH_3)_2)-R_1$$

steht, wobei $R_1$ CN, $COOR_2$, OH oder $OR_3$ bedeutet, $R_2$ für $C_1$—$C_8$-Alkyl steht und $R_3$ für Tosyl, Mesyl oder $C_2$—$C_4$-Alkanoyl steht, durch Alkylierung eines *p*-substituierten Phenols der Formel

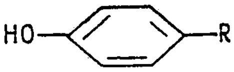

(II)

wobei R die oben angegebene Bedeutung hat, mit überschüssigem Chlordifluoromethan bei atmosphärischem oder überatmosphärischem Druck in Gegenwart einer Base, Wasser und einem inerten, mit Wasser mischbaren organischen Lösungsmittel oder Lösungsmittelgemisch, allein oder in Gegenwart von benzyltriethylammoniumchlorid, un eine difluoromethoxyaromatische Verbindung der Formel (I) zu erhalten, dadurch gekennzeichnet, daß Das Lösungsmittel ein Gemisch von 2-Propanol:Acetonitril oder 2-Propanol:Aceton im Volumenverhältnis von 1:1 bis 1:3 ist, und ferner gekennzeichnet dadurch, daß das Lösungsmittelgemisch in Mengen von 4 bis 6 ml pro Gramm der Verbindung der Formel (II) verwendet wird, wobei zu Beginn von etwa 870 bis 1740 Mol-% Wasser anwesend sind, mit zwei bis drei molaren Äquivalenten zugesetztem Chlordifluormethan.

2. Verfahren gemäß Anspruch 1, dadurch gekennzeichnet, daß die Reaktion bei einer Temperatur von etwa 20 bis 40°C durchgeführt wird.

3. Verfahren gemäß einem der Ansprüche 1 oder 2, dadurch gekennzeichnet, daß die Reaktion unter einem Druck von 0,4 bis 2,5 $kg/cm^2$ durchgeführt wird während eines Zeitraums im Bereich von etwa 30 Minuten bis 3 Stunden und mit einer wässrigen Lösung von vier molaren Äquivalenten eines Alkalimetallhydroxids von Natrium- oder Kalium-hydroxid, wobei die Konzentration der wässrigen Lösung derart ist, daß bei Beendigung der Basenzugabe die Gesamtmenge des zugesetzten Wassers in der Reaktionsmischung von etwa 1740 bis 2610 Mol-% beträgt und die Zugabe im wesentlichen gleichzeitig mit der chlordifluoromethan-Zugabe über einen Zeitraum im Bereich von etwa 1,0 bis 5,0 Stunden erfolgt; und wobei nach Beendigung der Basenzugabe das Reaktionsgemisch weiter bei den obigen Druck- und Temperaturbereichen gehalten wird während einer Zeitspanne von 0 bis 6 Stunden oder bis die Reaktion im wesentlichen vollständig ist.

4. Verfahren gemäß einem der Ansprüche 1 bis 3, dadurch gekennzeichnet, daß der Reaktion gegebenenfalls eine Hydrolyse des so hergestellten Esters folgt durch Erhitzen desselben mit einer starken Base in wasserfreiem Methanol, anschließende Entfernung des Lösungsmittels aus dem Reaktionsgemisch, Auflösen des Rückstands in Wasser und Behandlung der resultierenden Lösung mit starker Mineralsäure, um die 2-(*p*-Difluoromethoxyphenyl)-3-methylbuttersäure zu erhalten, und Vermischen von einem molaren Äquivalent der racemischen 2-(*p*-Difluoromethoxyphenyl)-3-methylbuttersäure in Gegenwart einer Mischung von Wasser und einem wasserunmischbaren Lösungsmittel mit einem Bereich der Gewichtsverhältnisse von 40/60 bis 60/40 mit einem optisch aktiven Amin, das in ausreichenden Mengen verwendet wird zur Bildung eines Salzes mit der rechtsdrehenden Komponente der racemischen Säure und einer anorganischen wasserlöslichen Base, die in ausreichenden Mengen verwendet wird, um die Gesamtmenge der genannten Base und des obigen Amins auf mindestens 100 Mol-% zu bringen; Erhitzen des so erhaltenen Gemisches auf eine Temperatur im Bereich von 40°C bis zum Siedepunkt der Mischung während einer ausreichenden Zeitspanne zur Bildung des optisch aktiven Aminsalzes der rechtsdrehenden Säure und Isolierung des Salzes vom dem Rest der Reaktionsmischung und Zersetzung desselben mit einer Mineralsäure, die in ausreichenden Mengen verwendet wird, um die rechtsdrehende Säure zu regenerieren.

5. Verfahren gemäß einem der Ansprüche 1 bis 3, dadurch gekennzeichnet, daß der Umsetzung gegebenenfalls eine Hydrolyse des so hergestellten Esters folgt durch Erhitzen desselben mit einer starken Base in wasserfreiem Methanol, anschließende Entfernung des Lösungsmittels von dem Reaktionsgemisch, Auflösen des erhaltenen Rückstands in Wasser und Behandlung der so erhaltenen Lösung mit Chlorwasserstoffsäure, um die 2-(*p*-Difluoromethoxyphenyl)-3-methylbuttersäure zu erhalten, und Vermischen von einem molaren Äquivalent racemischer 2-(*p*-Difluormethoxyphenyl)-3-methylbuttersäure in Gegenwart einer Mischung von Wasser und einem wasserungsmischbaren Lösungmittel, ausgewählt aus Benzol, Toluol und Xylol, und mit einem Bereich der Gewichtsverhältnisse von 40/60 bis 60/40, mit einem optisch aktiven Amin, ausgewählt aus (−)-α-Phenethylamin, (+) und (−)-2-Amino-1-butanol, Dehydroabiethylamin, (+) und (−)-α-4-Bromphenylethylamin, (−)-α-(1-Naphthyl)ethylamin, *l*-Ephedrin, 1*S*-2*S*-(+)-2-Amino-1-phenyl-1,3-propandiol, (+) und (−)-2-(4-Chlorbenzylamino)-1-butanol, (+) und (−)-2-(4-Thiobenzylamino)-1-butanol, (+) und (−)-2-(3-Nitrobenzylamino)-1-butanol, oder (+) und (−)-2-(2,5-Dimethylbenzylamino)-1-butanol, verwendet in Mengen von 40 bis 100 Mol-%; verwendent in Mengen ausreichen zur Bildung eines Salzes mit der rechtsdrehenden Komponente der genannten racemischen Säure, und eine anorganische wasserlösliche Base, ausgewählt aus Alkalimetallhydroxiden, Carbonaten oder Ammoniumhydroxid, verwendet in Mengen, die ausreichen, um die Gesamtmenge der Base und des obigen Amins auf mindestens 100 Mol-% zu bringen; Erhitzen des so erhaltenen Gemisches bei einer Temperatur im Bereich von 40°C bis zum Siedepunkt der Mischung während einer Zeitspanne, die ausreicht, um das optisch aktive Aminsalz der erwähnten rechtsdrehenden Säure zu bilden, Isolierung des Salzes von dem Rest der Reaktionmischung und Zersetzung desselben mit einer Mineralsäure, die in ausreichenden Mengen verwendet wird, um die erwähnte rechtsdrehende Säure daraus zu regenerieren; Ansäuern der restlichen Portion des oben erwähnten Reaktionsgemisches, enthaltend das Aminsalz der linksdrehenden (−)-Säure mit einer Mineralsäure, um die linksdrehende (−)-Säure zu erhalten, Veresterung der (−)-Säure unter Verwendung eines zweckentsprechenden Alkohols und einer

katalytischen Menge einer starken Mineralsäure, um den korrespondierenden (−)-Ester von (−)-2-(4-Difluormethoxyphenyl)-3-methylbuttersäure zu erhalten, Umsetzung von einem molaren Äquivalent des erwähnten $C_1$—$C_8$-Alkylesters von (−)-2-(4-Difluormethoxyphenyl)-3-methylbuttersäure mit einem bis zwei molaren Äquivalenten Natrium- oder Kaliumhydroxid in Anwesenheit von wasserfreiem $C_1$—$C_3$-Alkohol bei einer Temperatur von 60 bis 100°C oder beim Siedepunkt des gewählten Alkohols während einer Zeitspanne von 2 bis 10 Stunden oder bis die Reaktion im wesentlichen vollständig ist und sich das racemische Kaliumsalz der 2-[4-(Difluormethoxyphenyl)]-3-methylbuttersäure gebildet hat, Abtrennung des Lösungsmittels von der Reaktionsmischung, Auflösen des Rückstands in Wasser, Ansäuren der wässrigen Lösung mit starker Mineralsäure, un die racemische 2-[4-(difluormethoxyphenyl)]-3-methylbuttersäure zu erhalten und Rückführung derselben zum Zwecke ihrer Resolution.

6. Verfahren gemäß Anspruch 1, wobei man zu einer Lösung bei einer Temperatur von 30 bis 35°C ein molares Äquivalent einer Verbindung der Formel (II) in einem Lösungsmittelgemisch von 2-Propanol:Aceton, wobei das Volumenverhältnis des erwähnten Lösungsmittels 1:1 ist und in der Gesamtvolummenge von 4 ml/g einer Verbindung der Formel (I) und Wasser, welches zu Beginn mit der Rate von 1740 Mol-% zugesetzt ist, drei molare Äquivalente Chlordifluormethan zugibt über einen Zeitraum im Bereich von 30 Minuten bis 1,0 Stunden bei einem Druck von etwa 0,4 $kg/cm^2$ bis 1,1 $kg/cm^2$ zusammen mit einer initialen oder simultanen Zugabe von 4 molaren Äquivalenten einer wässrigen Natriumhydroxidlösung, welche über einen Zeitraum von etwa 1.0 bis 2.0 Stunden zugegeben wird und bei Beendigung der Basenzugabe die Gesamtmenge des zugesetzen Wassers 2610 Mol-% beträgt und die Reaktion weiter bei dem obigen Druck- und Temperaturbereich während einer Zeitspanne von etwa 1 bis 2 Stunden gehalten wird.

7. Verfahren gemäß Anspruch 1, wobei man drei molare Äquivalente Chlordifluormethan während eines Zeitraums von 30 Minuten bis 1,0 Stunden zugibt bei einem Druck von etwa 0,4 bis 1,1 $kg/cm^2$ bei einer Temperatur von etwa 30 bis 35°C und die obigen Druck- und Temperaturbereiche während etwa 1 bis 2 Stunden aufrechterhält; in Gegenwart von 2614 Mol-% Wasser, 1:1 2-Propanol:Aceton, das mit einer Rate von 4 ml/g der Verbindung der Formel (II) verwendet wird; das Alkalimetallhydroxid Natriumhydroxid ist; und die Gesamtmenge des dem Reaktionsgemisch zugesetzten Wassers 3482 Mol-% beträgt.

8. Verfahren gemäß Anspruch 1, wobei man 3 molare Äquivalente Chlordifluormethan während eines Zeitraums von etwa 30 Minuten bis 1,0 Stunden zusetzt bei einem Druck von etwa 0,4 bis 1,1 $kg/cm^2$ bei einer Temperatur von etwa 30 bis 35°C und die obigen Druck- und Temperaturbereiche während etwas 1 bis 2 Stunden Weiter Aufrechterhält; und wobei das Alkalimetallhydroxid Natriumhydroxid ist.

9. Verfahren gemäß einem der Ansprüche 4 bis 6, wobei die Verbindung Methyl-2-[4-(difluormethoxy)-phenyl]-3-methylbutyrat ist.

10. Verfahren gemäß einem der Ansprüche 4 bis 6, wobei die Verbindung Ethyl-2-[4-(difluormethoxy)-phenyl]-3-methylbutyrat ist.

11. Verfahren gemäß einem der Ansprüche 4 bis 6, wobei die Verbindung 2-[4-(Difluormethoxy)-phenyl]-3-methylbutyronitril ist.

12. Verfahren gemäß einem der Ansprüche 4 bis 6, wobei die Verbindung 4-Chlor-α,α-difluoranisol ist.

**Revendications**

1. Procédé de préparation d'un composé de formule développée:

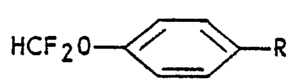

(I)

dans laquelle R représente un groupe alkyle en $C_1$—$C_3$, un atome d'halogène, un groupe nitro, ou R représente le groupe:

$$-CH(CH(CH_3)_2)-R_1$$

où $R_1$ représente —CN, —$COOR_2$, OH ou $OR_3$, $R_2$ représente un groupe alkyle en $C_1$—$C_8$ et $R_3$ représente un groupe tosyle, mésyle ou alcanoyle en $C_2$—$C_4$, par alkylation d'un phénol p-substitué de formule:

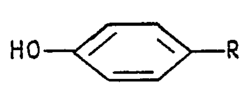

(II)

dans laquelle R est tel que décrit ci-dessus, avec un excès de chlorodifluorométhane à une pression supérieure ou égale à la pression atmosphérique, en présence d'un base, d'eau et d'un solvant organique inerte miscible dans l'eau ou d'un mélange de solvants, seul ou en présence de chlorure de benzyltriéthylammonium pour obtenir le composé difluorométhoxyaromatique de formule (I), caractérisé en ce que le solvant est un mélange de 2-propanol et d'acétonitrile, ou de 2-propanol et d'acétone suivant des rapports

en volume de 1:1 à 1:3, et caractérisé en outre en ce que ledit mélange de solvants est utilisé en des quantités de 4 à 6 ml par gramme du composé de formule (II) initialement en présence d'environ 870 à 1740 moles pour cent d'eau, et en ajoutant de 2 à 3 équivalents molaires de chlorodifluo-rométhane.

2. Procédé suivant la revendication 1, caractérisé en ce que la réaction est effectuée à une température d'environ 20 à 40°C.

3. Procédé suivant la revendication 1 ou 2, caractérisé en ce que la réaction est réalisée sous une pression de 0,4 à 2,5 kg.cm$^{-2}$ sur une période d'environ 30 minutes à houres et avec une solution aqueous de 4 équivalents molaires d'un hydroxyde de métal alcalin que est l'hydroxyde de sodium ou de l'hydroxyde potassium, en ce que la concentration de ladite solution aqueuse est telle qu'à la fin de l'addition de la base, la quantité totale d'eau ajoutée dans le mélange réactionnel est d'environ 1740 à 2610 moles pour cent, en étant ajoutée à peu près en même temps que l'addition de chlorodifluorométhane sur une période d'environ 1,0 à 5,0 heures; et en ce qu'à la fin de l'addition de la base, on maintient encore le mélange réactionnel dans les plages de température et de pression précitées pendant une période de 0 à 6 heures, ou jusqu'à ce que ladite réaction soit pratiquement achevée.

4. Procédé suivant l'une quelconque des revendications 1 à 3, caractérisé en ce que la réaction est éventuellement suivie par l'hydrolyse de l'ester ainsi préparé en chauffant ce dernier avec une base forte dans du méthanol anhydre, on élimine ensuite le solvant du mélange réactionnel, on dissout le résidu dans de l'eau et on traite la solution résultante avec un acide minéral forte pour obtenir l'acide 2-(p-difluorométhoxyphényl)-3-méthylbutyrique et on mélange un équivalent molaire de l'acide 2-(p-difluoro-méthoxyphényl)-3-méthylbu-tyrique racémique en présence d'un mélange d'eau et d'un solvant immiscible à l'eau suivant, des rapports en poids de 40/60 à 60/40, avec une amine optiquement active utilisée en des quantités suffisantes pour former un sel avec la composant dextrogyre dudit acide racémique et une base inorganique soluble dans l'eau utilisée en des quantitées suffisantes pour que la somme totale de ladite base et de l'amine précitée soit au moins de 100 moles pour cent; on chauffe le mélange ainsi obtenu à une température de 40°C jusqu'au point d'ébullition dudit mélange pendant une période suffisante pour former le sel d'amine optiquement actif dudit acide dextrogyre, on isole ledit sel du reste du mélange réactionnel et on le décompose un acide minéral utilisé en des quantités suffisantes pour régénérer ledit acide dextrogyre à partir de celui-ci.

5. Procédé suivant l'une quelconque des revendications 1 à 3, caractérisé en ce que la réaction est éventuellement suivie par l'hydrolyse de l'ester ainsi préparé en chauffant ce dernier avec une base forte dans du méthanol anhydre, on élimine ensuite le solvant du mélange réactionnel, on dissout le résidu de celui-ci dans de l'eau et on traite la solution ainsi obtenue avec de l'acide chlorhydrique pour obtenir l'acide 2-(p-difluorométhoxyphényl)-3-méthylbutyrique et on mélange un équivalent molaire de l'acide 2-(p-difluorométhoxyphényl)-3-méthylbutyrique en présence d'un mélange d'eau et d'un solvant immiscible à l'eau choisi parmi le benzène, le toluène et le xylène suivant des rapports de 40/60 à 60/40, avec une amine optiquement active choisie parmi la (−)-α-phénéthylamine, le (+) et la (−)-2-amino-1-butanol, la 1-déshydroabiéthylamine, la (+) et la (−)-4-bromophényléthylamine, la (−)-α-(1-naphtyl) éthyl-amine, la 1-éphédrine, les 1S-2S-(+)-2-amino-1-phényl-1,3-propanediol, le (+) et le (−)-2-(4-chloro-benzylamino)-1-butanol, le (+) et le (−)-2-(4-thio-benzylamino)-1-butanol, le (+) et le (−)-2-(3-nitrobenzylamino)-1-butanol ou le (+) et le (−)-2-(2,5-diméthylbenzylamino)-1-butanol, utilisé en des quantités de 40 à 100 moles pour cent suffisantes pour former un sel avec le composant dextrogyre dudit acide racémique, et une base inorganique soluble dans l'eau choisi parmi les hydroxydes, et les carbonates de métaux alcalins et l'hydroxyde d'ammonium, utilisée en des quantités suffisantes pour que la totalité de ladite base et de l'amine précitée soit d'eau moins 100 moles pour cent; on chauffe le mélange ainsi obtenu à une température de 40°C jusqu'au point d'ébullition dudit mélange pendant une période suffisante pour former le sel d'amine optiquement actif dudit acide dextrogyre, on isole ledit sel du reste du mélange réactionnel et on le décompose avec un acide minéral utilisé en des quantités suffisantes pour régénérer ledit acide detrogyre à partir de celui-ci; ou acidifie la portion restante dudit mélange réactionnel précité contenant le sel d'amine de l'acide (−) lévogyre avec un acide minéral pour obtenir l'acide (−) lévogyre; on estérifie ledit acide (−) en utilisant un alcool approprié et une quantité catalytique d'un acide minéral fort pour obtenir l'ester (−) correspondant de l'acide (−)-2-(4-difluorométhoxyphényl)-3-méthylbutyrique; on fait réagir un équivalent molaire dudit ester alkylique en $C_1$—$C_8$ de l'acide (−)-2-(4-difluorométhoxyphényl)-3-méthyl-butyrique avec un ou deux équivalents molaires d'hydroxyde de sodium ou de potassium en présence d'un alcool anhydre en $C_1$—$C_3$ à une température de 60 à 100°C ou au point d'ébullition de l'alcool choisi, pendant une période de 2 à 10 heures ou jusqu'à ce que la réaction soit à peu près achevée et que le sel recémique de potassium de l'acide -2-(4-difluorométhoxyphényl)-3-méthylbutyrique soite formé; on sépare le solvant du mélange réactionnel; on dissout le résidu restant dans l'eau; on acidifie la solution aqueuse avec un acide minéral fort pour obtenir l'acide -2-(4-difluorométhoxyphényl)-3-méthylbutyrique racémique et on recycle ce dernier pour le résoudre.

6. Procédé suivant la revendication 1, dans lequel on ajoite, dans une solution à une température de 30 à 35°C d'un équivalent molaire d'un composé de formule (II) dans un mélange de solvants constitué de 2-propanol et d'acétone dans lequel le rapport en volume desdits solvants est de 1:1 et suivant le quantité totale en volume de 4 ml par gramme de composé de formule (II) et d'eau qui est ajoutée initialement à raison de 1740 moles pour cent, 3 équivalents molaires de chlorodifluorométhane sur une période 30 minutes à 1,0 heure à une pression d'environ 0,4 kg.cm$^{-2}$ à 1,1 kg.cm$^{-2}$ initialement en combinaison avec

l'addition simultanée de 4 équivalents molaires d'une solution aqueuse d'hydroxyde de sodium qui est ajoutée sur une période d'environ 1,0 heure à 2,0 heures, et à la fin de l'addition de la base, la quantité totale d'eau ajoutée est de 2610 moles pour cent, et dans lequel on poursuit ensuite la réaction dans les plages de pression et de température précitées pendant une période d'environ 1 à 2 heures.

7. Procédé suivant la revendication 1, dans lequel on ajoute 3 équivalents molaires de chlorodifluorométhane en 30 minutes à 1,0 heure sous une pression d'environ 0,4 à 1,1 $kg.cm^{-2}$, à une température d'environ 30 à 35°C et on maintient ensuite les plages de pression et de température précitées pendant environ 1 à 2 heures, en présence de 2614 moles pour cent d'eau, d'un mélange 1:1 de 2-propanol et d'acétone utilisé à raison de 4 ml par gramme du composé de formule (II); l'hydroxyde de métal alcalin étant de l'hydroxyde de sodium et la quantité totale d'eau ajoutée dans la mélange réactionnel étant de 3482 moles pour cent.

8. Procédé suivant la revendication 1, dans lequel on ajoute 3 équivalents molaires de chlorodifluorométhane en environ 30 minutes à 1,0 heure à une pression d'environ 0,4 à 1,1 $kg.cm^{-2}$, à une température d'environ 30 à 35°C, et dans lequel on maintient ensuite les plages de pression et de température précitées pendant environ 1 à 2 heures; l'hydroxyde de métal alcalin étant de l'hydroxyde de sodium.

9. Procédé suivant l'une quelconque des revendications 4 à 6, dans lequel ledit composé est le 2-[4-(difluorométhoxy)phenyl]-3-méthylbutyrate de méthyle.

10. Procédé suivant l'une quelconque des revendications 4 à 6, dans lequel ledit composé est le 2-[4-(difluorométhoxy)phenyl]-3-méthylbutyrate de éthyle.

11. Procédé suivant l'une quelconque des revendications 4 à 6, dans lequel ledit composé est le 2-[4-(difluorométhoxy)phenyl]-3-méthylbutyronitrile.

12. Procédé suivant l'une quelconque des revendications 4 à 6, dans lequel ledit composé est le 4-chloro-α-α-difluoroanisole.